# EUROPÄISCHE PATENTSCHRIFT

(11) **EP 1 309 297 B1**
(45) Veröffentlichungstag und Bekanntmachung des Hinweises auf die Patenterteilung: **02.11.2005**
(21) Anmeldenummer: 01976083.4
(22) Anmeldetag: 16.08.2001
(51) Int. Cl.: A61F 2/78, A61F 2/64

(54) **BEINPROTHESE ZUR ADAPTION AN EINEN OBERSCHENKELSTUMPF**
BONE PROSTHESIS FOR ADAPTING TO A FEMORAL STUMP
PROTHESE DE LA JAMBE A ADAPTER A UN MOIGNON DE FEMUR

(30) Priorität: 16.08.2000 DE 10040617
(43) Veröffentlichungstag der Anmeldung: 14.05.2003
(73) Patentinhaber: ESKA Implants GmbH & Co., 23556 Lübeck (DE)
(72) Erfinder: GRUNDEI, Hans, 23556 Lübeck (DE)
(74) Vertreter: Fuchs Mehler Weiss & Fritzsche
(86) Internationale Anmeldenummer: PCT/EP2001/009433
(87) Internationale Veröffentlichungsnummer: WO 2002/013733

(56) Entgegenhaltungen:
- EP-A- 0 547 354
- DE-C- 19 845 191
- DE-C- 19 857 907
- US-A- 3 947 897

## Beschreibung

Die vorliegende Erfindung betrifft eine Beinprothese zur Adaption an einen Oberschenkelstumpf, bestehend aus einem intramedullär im Femurstumpf zu verankernden Adapter für ein extrakorporal ankoppelbares Kniegelenk und einer am Kniegelenk angekoppelten Unterschenkelprothese.

Eine derartige Beinprothese ist beispielsweise bekannt aus der DE-C-198 45 191.

Mit dieser Beinprothese wird das Ziel verfolgt, die sogenannte Osteoperzeption des oberschenkelamputierten Patienten entscheidend zu verbessern, dadurch, daß ein intramedulläres Stielteil des Adapters in den Femurstumpf eingesetzt wird und dort aufgrund einer offenmaschigen, dreidimensionalen Oberflächenstruktur mit dem natürlichen Knochenmaterial verwächst, also eine sehr innige Verbindung zwischen dem Implantat einerseits und dem natürlichen Knochen andererseits angestrebt wird.

Die Verbindung zwischen dem Adapter und dem natürlichen Knochen kann sich allerdings als so fest herausstellen, daß eben diese an sich gewünschte Festigkeit zu Problemen führen kann, wenn es nämlich um äußergewöhnliche Kraftbelastungen geht. So ist es nicht von vornherein auszuschließen, daß eine extreme, beispielsweise Scherbelastung des künstlichen Kniegelenkes oder eine Belastung der Unterschenkelprothese in den intramedullär verankerten Adapter im Femurstumpf weitergeleitet wird und dort zu Brüchen führen kann. Diese Situation ist für den Patienten nicht akzeptabel.

Im Rahmen der vorliegenden Erfindung gilt es daher, dieses Problem bei einer gattungsgemäßen Beinprothese zu lösen.

Diese Aufgabe wird erfindungsgemäß dadurch gelöst, daß eine extrakorporal angeordnete Sollbruchstelle in mindestens einem Prothesenteil der Beinprothese vorgesehen ist, welche durch Sollbrechen bei einer vorbestimmten Kraft das Prothesenteil abbrechen läßt.

Wesentlich hierbei ist, daß die Sollbruchstelle in einem der Prothesenteile extrakorporal anzuordnen ist, damit in einem Unglücksfalle keine inkorporalen Teile der Beinprothese bzw. Teile des Oberschenkelstumpfes in Mitleidenschaft geraten.

Gemäß einer vorteilhaften Ausführungsform ist vorgesehen, daß die Sollbruchstelle im Bereich der Ankopplung des Kniegelenks an den intramedullär verankerten Adapter angeordnet ist. Dies geschieht vorzugsweise durch Anordnung einer Sollbruchstelle in einem Zwischenstück zwischen Adapter und Kniegelenk, damit der dauerhaft implantierte Adapter selbst nicht in Leidenschaft gezogen wird, sollte es einmal aufgrund außergewöhnlicher Kraftbelastungen zum Aufbruch der Sollbruchstelle kommen.

Gemäß einer anderen Ausführungsform ist vorgesehen, daß die Sollbruchstelle im Bereich einer Seitenführung des Kniegelenks angeordnet ist. Die Seitenführung bildet dabei die Funktion der natürlichen Seitenbänder ab. Sollte es aufgrund spezifischer Belastungen zu einer Überhöhung der zulässigen Belastung kommen, so würde die im Bereich der Seitenführung vorgesehene Sollbruchstelle für eine schlagartige Entlastung der übrigen Beinprothese kommen.

Alternativ hierzu ist eine Ausführungsform vorgesehen, bei der die Sollbruchstelle im Bereich der Ankopplung des Kniegelenks an die Unterschenkelprothese angeordnet ist.

Die Entscheidung hierüber, wo die Sollbruchstelle tatsächlich angeordnet wird, hängt unter anderem beispielsweise davon ab, welche Teile besonders kostspielig sind und daher möglichst nicht einem Aufbrechen unterworfen werden sollen. Bei einem einfachen Scharniergelenk als Kniegelenk mag die Anordnung der Sollbruchstelle im Bereich der Ankopplung des Kniegelenks an die Unterschenkelprothese wünschenswert sein. Bei Einsatz eines relativ aufwendigen Kniegelenks, beispielsweise wie es sich aus der EP-0 358 056 B1 ergibt, käme sicherlich die Anordnung der Sollbruchstelle im Bereich der Ankopplung des Kniegelenks an den Adapter in Betracht.

Besonders bevorzugt ist die Ausführung der Sollbruchstelle als Materialschwächung in dem betroffenen Bereich. Dies ist durch eine relativ einfache Maßnahme, wie eine zusätzliche Ausfräsung, relativ einfach zu realisieren.

Es ist jedoch gemäß einer vorteilhaften Weiterbildung auch möglich, mehrere Sollbruchstellen in unterschiedlichen Prothesenteile vorzusehen, die jeweils so ausgeführt sind, daß jede einzelne Sollbruchstelle auf jeweils eine andere spezifische Belastung anspricht. So ist es möglich, beispielsweise im Bereich einer Seitenführung des Kniegelenks eine Sollbruchstelle vorzusehen, die bei extremen lateral-medialen Beanspruchungen anspricht, d. h. abbricht, und zum anderen eine Sollbruchstelle im Bereich der Ankopplung des Kniegelenks an den Adapter, welche beispielsweise auf dorsal-ventrale Belastungen anspricht.

Vorteilhaft ist es, die Sollbruchstellen so auszuführen, daß diese bei Belastungskräften im Bereich von 150 bis 500 Kp abbrechen. Dies simuliert in etwa die Kräfte, die in einem natürlichen Bein Reaktionen beispielsweise im Kniebereich auslösen, bietet jedoch noch hinreichend Reserve vor dem Hintergrund der spezifischen Probleme der Beinprothese, bei welcher eben eine Belastung im Knie oder Unterschenkelbereich zu einem Bruch im Oberschenkelbereich führen kann aufgrund der intramedullären Verankerung des erwähnten Adapters.

Die Erfindung wird anhand eines Ausführungsbeispiels gemäß der Zeichnungsfiguren näher erläutert. Hierbei zeigt:
- Fig. 1: einen schematischen Überblick über die Beinprothese, und
- Fig. 2: eine Vergrößerung der Ansicht der Beinprothese im Bereich des Kniegelenkes der Prothese aus Fig. 1.

Nachfolgend sind die gleichen Teile mit denselben Bezugszeichen bezeichnet.

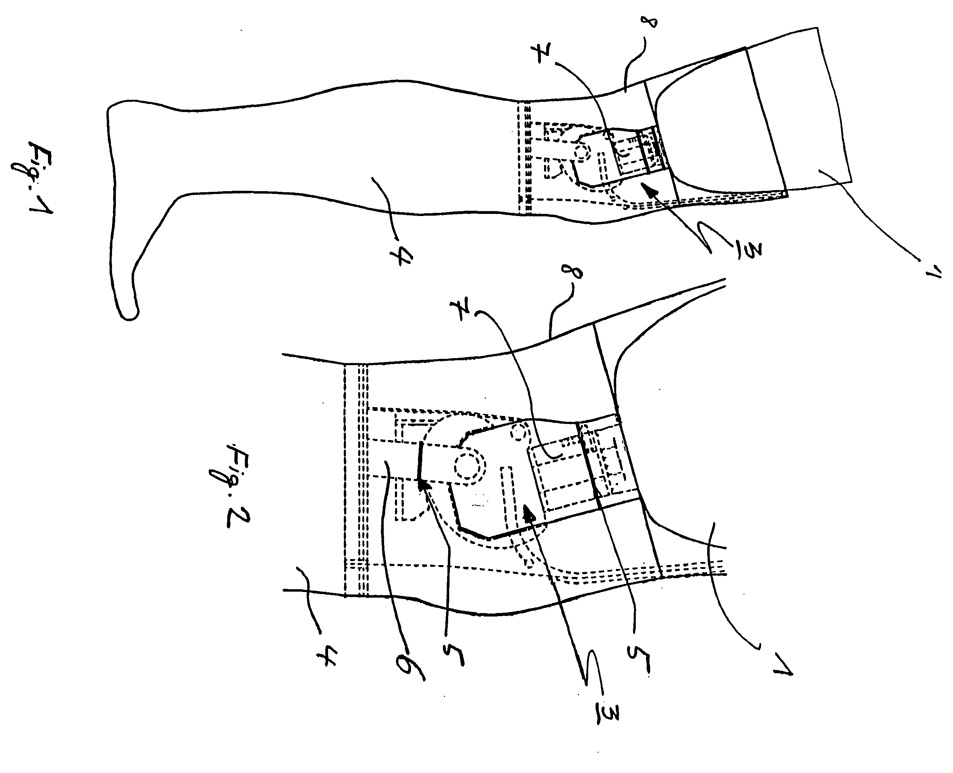

Den ersten Überblick verschafft Fig. 1. Mit dem Oberschenkel I ist über ein intramedullär verankerter Adapter 7 ein künstliches Kniegelenk 3 verbunden, welches seinerseits mit einer Unterschenkelprothese 4 gekoppelt ist. Der Übergangsbereich zwischen Unterschenkelprothese 4 und dem Oberschenkelstumpf 1 ist bedeckt mit einer Schutzmanschette 8.

Wie aus Fig. 2 hervorgeht, ist im Bereich der Ankopplung des Kniegelenks 3 an den Adapter 7 eine Sollbruchstelle 5 in Form einer Materialschwächung des am Adapter 7 angeflanschten Teils des Kniegelenks 3 vorgesehen. Diese Sollbruchstelle kann beispielsweise durch eine Ausfräsung, d. h. eine Materialschwächung des angeflanschten Teils realisiert sein, vorzugsweise so, daß diese bei auftretenden Belastungskräften in Höhe von 150 bis 500 Kp abbricht, so daß derartige Belastungen über den Adapter 7 nicht in den Oberschenkelstumpf 1, und dort insbesondere in den Femurstumpf eingeleitet werden.

In Ergänzung oder alternativ hierzu ist eine weitere Sollbruchstelle 5 in einer Seitenführung 6 des speziell ausgebildeten Kniegelenks 3 vorgesehen. Diese Sollbruchstelle ist insbesondere dafür vorgesehen, bei einen bestimmten Grenzwert überschreitenden lateral-medialen Belastungskräfte aufzubrechen, um Schaden vom Femurstumpf abzuwenden.

Aufgrund der Kombination mehrfacher Sollbruchstellen läßt sich ein individuelles Bruchverhalten der Beinprothese realisieren.

## Patentansprüche

1. Beinprothese zur Adaption an einen Oberschenkelstumpf (1), bestehend aus einem intramedullären im Femurstumpf zu verankernden Adapter für ein extrakorporal anzukoppelndes Kniegelenk (3) und einer am Kniegelenk (3) ankoppelbaren Unterschenkelprothese (4), **gekennzeichnet durch eine** extrakorporal angeordnete Sollbruchstelle (5) in mindestens einem Prothesenteil (3; 4), welche **durch** Applikation einer Kraft vorbestimmter Größe das Prothesenteil (3; 4) abbrechen läßt.

2. Beinprothese nach Anspruch 1, **dadurch gekennzeichnet, daß** die Sollbruchstelle (5) im Bereich der Ankopplung des Kniegelenks (3) an den Adapter (7) angeordnet ist.

3. Beinprothese nach Anspruch 1, **dadurch gekennzeichnet, daß** die Sollbruchstelle (5) im Bereich einer Seitenführung (6) des Kniegelenks (3) angeordnet ist.

4. Beinprothese nach Anspruch 1, **dadurch gekennzeichnet, daß** die Sollbruchstelle (5) im Bereich der Ankopplung des Kniegelenks (3) an die Unterschenkelprothese (4) angeordnet ist.

5. Beinprothese nach einem der Ansprüche 1 bis 4, **dadurch gekennzeichnet, daß** die Sollbruchstelle (5) durch Materialschwächung des bettvffenen Bereiches ausgeführt ist.

6. Beinprothese nach einem der Ansprüche 1 bis 5, **dadurch gekennzeichnet, daß** mehrere Sollbruchstellen (5) so angeordnet und ausgeführt sind, daß jede einzelne Sollbruchstelle auf eine jeweils andere Belastung anspricht und abbricht.

7. Beinprothese nach einem der Ansprüche 1 bis 6, **dadurch gekennzeichnet, daß** die Sollbruchstelle (5) bei Belastungskräften im Bereich von 150 bis 500 Kp abbricht.

## Claims

1. Leg prosthesis for adaptation to a femoral stump (1) consisting of a intramedullary adapter to be anchored in the femoral stump for a knee joint (3) to be coupled in extracorporeal manlier and a lower leg prosthesis (4) which can be coupled to the knee joint (3). **characterised by** a predetermined breaking point (5) arranged in extracorporeal manner in at least one prosthesis part (3; 4). which allows the prosthesis part (3:4) to break off by application of a force of predetermined size.

2. Leg prosthesis according to claim 1, **characterised in that** the breaking point (5) is arranged in the region of the coupling of the knee joint (3) to the adapter (7).

3. Leg prosthesis according to claim 1, **characterised in that** the breaking point (5) is arranged in the region of a side guide (6) of the knee joint (3).

4. Leg prosthesis aceordin8 to claim 1, **characterised in that** the breaking point (5) is arranged in the region of the coupling of the knee joint (3) to the lower leg prosthesis (4).

5. Leg prosthesis according to one of claims 1 to 4, **characterised in that** the breaking point (5) is implemented by material weakening of the region concerned.

6. Leg prosthesis according to one of claims 1 to 5, **characterised in that** several breaking points (5) are arranged and implemented so that each individual breaking point responds and breaks off in each ease to a different load.

7. Leg prosthesis according to one of claims 1 to 6, **characterised in that** the breaking point (5) breaks off at load forces in the range from 150 to 500 Kp.

## Revendications

1. Prothèse de jambe à adapter à un moignon de cuisse (1), composée d'un adaptateur intramédullaire à ancrer dans le moignon du fémur pour une articulation de genou (3) à coupler de manière extracorporelle et d'une prothèse de jambe (4) à coupler à l'articulation de genou (3), **caractérisée par** un point de rupture (5) disposé de manière extracorporelle dans au moins une partie de la prothèse (3, 4), qui entraîne la rupture de la prothèse (3, 4) suite à l'application d'une force de grandeur prédéterminée.

2. Prothèse de jambe selon la revendication 1, **caractérisée en ce que** le point de rupture (5) est disposé dans la zone du couplage de l'articulation de genou (3) à l'adaptateur (7).

3. Prothèse selon la revendication 1, **caractérisée en ce que** le point de rupture (5) est disposé dans la zone d'un guidage latéral (6) de l'articulation de genou (3).

4. Prothèse de jambe selon la revendication 1, **caractérisée en ce que** le point de rupture (5) est disposé dans la zone du couplage de l'articulation de genou (3) à la prothèse de jambe (4).

5. Prothèse de jambe selon une des revendications 1 à 4, **caractérisée en ce que** le point de rupture (5) est réalisé par l'affaiblissement du matériau de la zone concernée.

6. Prothèse de jambe selon une des revendications 1 à 5, **caractérisée en ce que** plusieurs points de rupture (5) sont disposés et réalisés de telle manière que chaque point de rupture réagisse et se rompe sous l'effet d'une autre charge.

7. Prothèse de jambe selon une des revendications 1 à 6, **caractérisée en ce que** le point de rupture (5) se rompt sous l'effet de forces de charge de 150 à 500 kp.
